# EUROPÄISCHE PATENTSCHRIFT

(11) **EP 1 703 937 B1**
(45) Veröffentlichungstag und Bekanntmachung des Hinweises auf die Patenterteilung: **30.09.2009**
(21) Anmeldenummer: 04803444.1
(22) Anmeldetag: 02.12.2004
(51) Int. Cl.: A61M 25/01

(54) **VORRICHTUNG ZUR HANDHABUNG EINES KATHETERS**
DEVICE FOR OPERATING A CATHETER
DISPOSITIF POUR MANIPULER UN CATHETER

(30) Priorität: 08.01.2004 DE 102004001461
(43) Veröffentlichungstag der Anmeldung: 27.09.2006
(73) Patentinhaber: Wollschläger, Helmut, 90480 Nürnberg (DE)
(72) Erfinder: Wollschläger, Helmut, 90480 Nürnberg (DE)
(74) Vertreter: Lindner, Michael
(86) Internationale Anmeldenummer: PCT/EP2004/013701
(87) Internationale Veröffentlichungsnummer: WO 2005/065762

(56) Entgegenhaltungen:
- WO-A-01/15768
- DE-C1- 19 526 075
- DE-C1- 19 948 409
- US-A- 5 968 052

## Beschreibung

Die Erfindung betrifft eine Vorrichtung zur Handhabung eines Katheters mit einem länglichen Ventilkörper, einem Hebelarm, der über ein Federelement vorgespannt ist und an dem Ventilkörper schwenkbar angebracht ist, um aus einer ersten Endposition gegen die Vorspannungskraft des Federelements in eine zweite Endposition schwenkbar zu sein, einem im Ventilkörper aufgenommenen Druckkolben, der über den Hebelarm in Längsrichtung bewegbar ist, um ein Dichtungselement im Ventilkörper bei einer Bewegung des Hebelarms in Richtung der zweiten Endposition zu öffnen, und einem Rastmechanismus, der eine Arretiereinrichtung und eine damit zusammenwirkende Rastzunge aufweist, um den Hebelarm in zumindest einer Raststellung zu halten.

Eine derartige Vorrichtung ist beispielsweise aus der DE 195 26 075 C1 bekannt. Bei dieser Vorrichtung ist zur Betätigung eines in einem Ventilkörper eingeführten Druckkolbens über einen Hebelarm an einem Ventilkörper einstückig ein biegsames Arretierstück mit einer Rastnase ausgebildet, mit dem ein Rastende des Hebelarms in einer Raststellung haltbar ist. Das Arretierstück erstreckt sich im Wesentlichen rechtwinklig zu dem Ventilkörper. In der Raststellung wird auf einen durch den Ventilkörper und den Druckkolben durchgeführten Katheter eine Klemmkraft ausgeübt, um den Katheter gegen unbeabsichtigte Verschiebung in axialer Richtung zu sichern.

Eine weitere derartige Vorrichtung ist beispielsweise aus WO 01/15768 A1 bekannt. Bei dieser Vorrichtung weist das Arretierstück mehrere Rastnasen zur Verrastung des Hebelarms in mehreren Raststellungen auf und verfügt das Arretierstück über einen Betätigungsflügel, der in den Raststellungen des Hebelarms in Verlängerung des Hebelarms ausgerichtet ist.

Obgleich sich die vorgenannte Vorrichtung sehr einfach und ergonomisch bedienen lässt, besteht seitens der Nutzer der Wunsch, die Bedienbarkeit weiter zu verbessern.

Vor diesem Hintergrund besteht die Aufgabe der vorliegenden Erfindung darin, die eingangs genannte Vorrichtung so weiterzubilden, dass eine einfachere Bedienung ermöglicht wird.

Diese Aufgabe wird bei der vorgenannten Vorrichtung dadurch gelöst, dass der Rastmechanismus so ausgebildet ist, dass die Arretiereinrichtung und die Rastzunge bei Erreichen der zweiten Endposition des Hebelarms außer Eingriff gelangen, so dass das Federelement den Hebelarm zurück in die erste Endposition führt.

D.h. mit anderen Worten, dass der Rastmechanismus so ausgelegt ist, dass der Nutzer den Hebelarm lediglich in die zweite Endposition drücken muss, um die erste Endposition wieder zu erreichen. Erreicht der Hebelarm die zweite Endposition, löst sich die Rastzunge von der Arretiereinrichtung und gibt sozusagen den Rastmechanismus frei, so dass sich der Hebelarm dann in die erste Endposition bewegen kann. Für die Rückführung des Hebelarms in die erste Endposition sorgt dabei das Federelement.

Die Bedienung ist für den Nutzer deshalb so einfach, weil er letztlich nur eine Bewegung des Hebelarms ausführen muss, nämlich das Drücken des Hebelarms in Richtung der zweiten Endposition. Es ist folglich nicht mehr notwendig, beispielsweise durch Drücken eines Betätigungsflügels die Raststellung des Hebelarms zu lösen, wie dies in der oben genannten Druckschrift WO 01/15768 A1 vorgeschlagen ist.

In einer bevorzugten Weiterbildung der Erfindung weist die Arretiereinrichtung mehrere Rastnasen auf, die entlang einer konzentrischen Linie um den Drehpunkt des Hebelarms angeordnet sind, um mehrere Raststellungen des Hebelarms zwischen den beiden Endpositionen zu definieren.

Diese Maßnahmen haben den Vorteil, dass die mehreren Raststellungen die Bedienbarkeit weiter vereinfachen, da der Nutzer die auf den Katheter wirkende Klemmkraft besser einstellen kann.

In einer weiteren bevorzugten Ausführungsform folgt der dem Ventilkörper zugewandten Rastnase eine schräg verlaufende Führungsfläche, um bei Erreichen der zweiten Endposition des Hebelarms die Rastzunge auf die der Rastnase abgewandte Seite der Arretiereinrichtung zu führen, so dass die Rastzunge bei der Rückführung des Hebelarms in die erste Endposition nicht mit den Rastnasen in Eingriff gelangen kann.

D.h. mit anderen Worten, dass an dem dem Ventilkörper zugewandten Ende der Arretiereinrichtung eine Fläche vorgesehen ist, die schräg nach oben (weg vom Ventilkörper) zu der bezüglich der Rastnasen rückwärtigen Fläche verläuft. Damit wird die Rastzunge am Hebelarm bei Erreichen der zweiten Endposition auf die Rückseite der Rastnasen geführt, so dass die Rastzunge beim Weg des Hebelarms in die erste Endposition nicht mit den Rastnasen in Kontakt kommen kann.

Diese Maßnahme führt zu einer sehr einfachen Ausgestaltung der Arretiereinrichtung und ermöglicht eine sehr sichere Bedienung.

In einer weiteren bevorzugten Ausführungsform erstrecken sich die Rastzunge und die Rastnasen quer zur Längsrichtung des Ventilkörpers.

Diese Maßnahme ist aus konstruktiver Sicht besonders einfach.

In einer bevorzugten Weiterbildung weist der Hebelarm ein Langloch auf, durch das sich ein vom Ventilkörper abgehendes Seitenrohr hindurch erstreckt.

Diese Maßnahme ermöglicht eine sehr kompakt bauende Vorrichtung mit hoher Stabilität, insbesondere auch im Hinblick auf den Haltearm, der durch das Zusammenwirken von Langloch und Seitenrohr zusätzlich stabilisiert werden kann.

Der Ventilkörper und/oder der Hebelarm sind bevorzugt als Spritzgießteile ausgebildet, wobei der Ventilkörper und die Arretiereinrichtung vorzugsweise als einstückige Bauteile vorgesehen sind.

Weitere Vorteile und Ausgestaltungen der Erfindung ergeben sich aus der Beschreibung und der beiliegenden Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nun an Hand einer bevorzugten Ausführungsform mit Bezug auf die Zeichnung beispielhaft erläutert. Dabei zeigen:
- Fig. 1: eine schematische Seitenansicht der erfindungsgemäßen Vorrichtung;
- Fig. 2: eine schematische Darstellung des Rastmechanismus;
- Fig. 3: eine schematische Draufsicht des Hebelarms; und
- Fig. 4A bis 4I: verschiedene Darstellungen des Rastmechanismus in verschiedenen Positionen, um dessen Funktionsweise zu beschreiben.

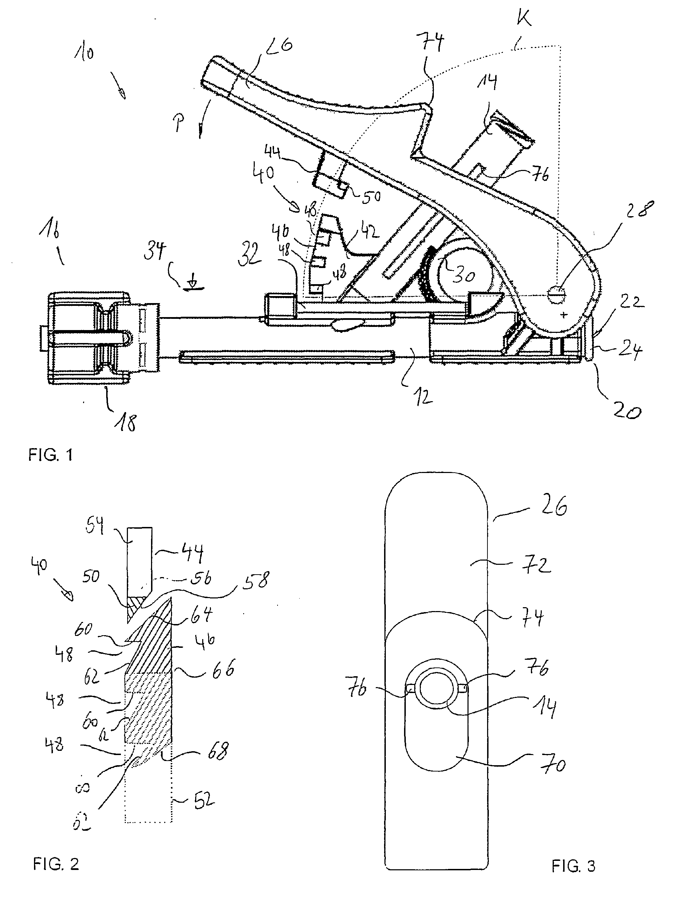

In Fig. 1 ist eine erfindungsgemäße Vorrichtung in schematischer Darstellung von der Seite gezeigt und mit dem Bezugszeichen 10 gekennzeichnet. Die Vorrichtung 10 weist einen länglichen Ventilkörper 12 auf, der eine nicht dargestellte axiale Ventilkörperdurchführung besitzt. Der Ventilkörper 12 stellt damit ein rohrförmiges Element dar, das an seinen beiden Enden offen ist. An dem Ventilkörper 12 ist in einem mittleren Längsbereich ein Seitenrohr 14 einstückig angesetzt, wobei sich das Seitenrohr 14 schräg zur Längsachse des Ventilkörpers, beispielsweise in einem Winkel von 45°, erstreckt. Das Seitenrohr 14 mündet dabei in die Ventilkörperdurchführung.

An einem bei bestimmungsgemäßer Verwendung der Vorrichtung 10 von dem Körper eines Patienten weg weisenden Anschlussende 16 ist eine Drehmanschette 18 vorgesehen, mit der in an sich bekannter Weise der Ventilkörper 12 bei einem eingeführten Führungskatheter abgedichtet verschließbar ist. In einem dem Anschlussende 16 gegenüberliegenden Ende 20, das bei bestimmungsgemäßer Verwendung der Vorrichtung 10 dem Körper eines Patienten zugewandt ist, ist eine Abdichteinrichtung 22 vorgesehen, die aus einem Druckkolben 24 und einem in der Ventilkörperdurchführung vorgesehenen und am inneren Ende des Druckkolbens 24 liegenden Dichtpfropfen besteht. Der in Fig. 1 nicht zu erkennende Dichtpfropfen ist aus einem elastischen Material gefertigt und besitzt eine in Längsrichtung durchgehende Öffnung, die unter Druckbelastung in Längsrichtung verschließbar ist. Diese Druckbelastung wird über den Druckkolben 24 auf den Dichtpfropfen aufgebracht.

Die Vorrichtung 10 weist ferner einen Hebelarm 26 auf, der um eine Achse 28 schwenkbar am Anschlussende 20 angebracht ist.

Der Hebelarm 26 wird über eine Drehfeder 30 in eine erste in der Fig. 1 gezeigte Endposition vorgespannt. Die mit schraubenförmigen Windungen versehene Drehfeder 30 besitzt hierfür einen am Ventilkörper 12 festgelegten Endabschnitt 32 und einen an einer dem Ventilkörper 12 zugewandten Fläche des Hebelarms 26 festgelegten Endabschnitt, der in der Figur jedoch nicht zu sehen ist.

Der Hebelarm 26 lässt sich aus der gezeigten ersten Endposition in Richtung des Pfeils P in eine zweite Endposition 34 gegen die Kraft der Drehfeder 30 um die Achse 28 drehen. Ohne entsprechende Belastung des Hebelarms 26 wird dieser über die Drehfeder 30 wieder in die erste gezeigte Endposition zurückgeführt.

An dem Hebelarm 26 ist ein in Fig. 1 nicht dargestelltes Bauteil vorgesehen, das mit dem Druckkolben 24 derart zusammenwirkt, dass der Druckkolben 24 bei einer Bewegung des Hebelarms 26 in die zweite Endposition 34 in Längsrichtung verlagert wird, um die Belastung auf den Dichtpfropfen zu reduzieren und damit die Durchführung im Dichtpfropfen zu öffnen. D.h. mit anderen Worten, dass der Druckkolben 24 bei vorgespanntem Hebelarm 26 in der ersten Endposition so stark gegen den Dichtpfropfen gedrückt wird, dass die Durchführung vollständig geschlossen ist.

Der Verwendungszweck einer solchen medizinischen Vorrichtung ist allgemein bekannt, so dass darauf nicht näher eingegangen werden soll. Mit wenigen Worten dient die Vorrichtung 10 dazu, einen durch den Ventilkörper 12 hindurch laufenden Katheter über den Dichtpfropfen abdichtend festzuklemmen, wobei die Klemmkraft durch Betätigung des Hebelarms 26 reduziert werden kann, um den Katheter in Längsrichtung bewegen zu können.

Im Übrigen wird bezüglich der Funktionsweise und des Aufbaus des Ventilkörpers 12 auf die WO 01/15768 verwiesen. Der Offenbarungsgehalt dieser Druckschrift wird in diesem Umfang durch Bezugnahme in die vorliegende Anmeldung mit aufgenommen.

Um den Hebelarm 26 in unterschiedlichen Stellungen halten zu können, ist ein Rastmechanismus 40 vorgesehen, der eine dem Ventilkörper 12 zugeordnete Arretiereinrichtung 42 und eine dem Hebelarm 26 zugeordnete Rastzungeneinrichtung 44 umfasst.

Die Arretiereinrichtung 42 ist als plattenförmiges Teil ausgebildet, das an dem Seitenrohr 14 angebracht ist. An dem dem Seitenrohr 14 abgewandten Randbereich 46 sind mehrere, bei der vorliegenden Ausführungsform beispielhaft drei, Rastnasen 48 vorgesehen.

Die Rastzungeneinrichtung 44 weist ihrerseits eine Rastzunge 50 auf, die mit den Rastnasen 48 zusammenwirken kann. Folglich liegen die Rastnasen 48 und die Rastzunge 50 auf einer konzentrischen Linie K um die Drehachse 28.

Die Rastzungeneinrichtung 44 ist an der dem Ventilkörper zugewandten Unterseite des Hebelarms 26 ebenfalls als streifen- bzw. plattenförmiges Element angebracht.

Der Rastmechanismus 40 ist in vergrößerter schematischer Darstellung in Fig. 1 nochmals getrennt gezeigt. Die Ansicht erfolgt hierbei von dem Anschlussende 20, so dass der plattenförmige Teil der Arretiereinrichtung 42, der mit dem Bezugszeichen 52 gekennzeichnet ist, einen Abschnitt des Randbereichs verdeckt. Dies ist durch die punktierte Linie und die etwas schwächere Schraffur des Randbereichs 46 dargestellt.

Die Rastzunge 50 der Rastzungeneinrichtung 44 ist beispielsweise als dreieckförmiges Bauteil ausgebildet, das gegenüber dem Grundkörper 54 der Rastzungeneinrichtung 44 hervorspringt. Mit Bezug auf die Zeichenebene in Fig. 2 liegt die Rastzunge 50 damit vor dem Grundkörper 54. Die Rastzunge 50 besitzt eine Rastfläche 56 und eine schräg dazu verlaufende Fläche 58. Die Rastfläche 56 verläuft etwa auf einer radialen Linie bezüglich der Drehachse 28.

Die Arretiereinrichtung 42 besitzt die genannten drei Rastnasen 48, die als dreieckförmige Ausnehmungen in dem Randbereich 46 ausgebildet sind. Die dreieckförmigen Ausnehmungen besitzen jeweils eine Rastfläche 60, die etwa parallel zu der Rastfläche 56 ausgebildet sind.

Die Abmessungen der Rastzunge 50 und der Rastnasen 48 sind so ausgelegt, dass die Rastfläche 56 möglichst vollflächig jeweils mit der Rastfläche 60 der drei Rastnasen 48 zusammenwirken kann. Um ein Lösen der Rastverbindung zu erleichtern, besitzt jede Rastnase 48 eine zu der Rastfläche 60 schräg nach links unten sich erstreckende Fläche 62. Diese Fläche 62 wirkt mit der schräg verlaufenden Fläche 58 der Rastzunge 50 derart zusammen, dass die Rastzungeneinrichtung 44 bei einer Bewegung nach unten aus der Rastnase 48 herausbewegt wird.

Um zu gewährleisten, dass die Rastzunge 50 bei einer Bewegung des Hebelarms 26 aus der ersten Endposition in die obere Rastnase 48 gelangt, weist der Randbereich eine schräg nach links (bezogen auf die in Fig. 2 gezeigte Darstellung) abfallende obere Kantenfläche 64 auf. Diese Kantenfläche 64 verhindert, dass die Rastnase 50 beim Bewegen des Hebelarms 26 aus der ersten Endposition auf die den Rastnasen 48 abgewandte Rückseite 66 des Randbereichs 46 gelangt.

Am unteren Ende des Randbereichs 46 ist ebenfalls eine Kantenfläche 68 vorgesehen, die (bezogen auf die Darstellung in Fig. 2) schräg nach links abfallend verläuft. Die Aufgabe dieser Kantenfläche 68 besteht darin, die Rastzungeneinrichtung 44 nach Überschreiten der untersten Rastnase 48 und Lösen der Betätigungskraft auf die Rückseite 66 zu führen. Hierbei gleitet die Rastfläche 56 der Rastzunge 50 an der Kantenfläche 68 entlang.

Mit Hilfe dieses Rastmechanismus ist es folglich möglich, den Hebelarm 26 in drei vorgegebenen Raststellungen zu verrasten und in die ursprüngliche erste Endposition zurückzubringen, indem der Hebelarm 26 in die untere zweite Endposition 34 gedrückt und dann losgelassen wird. Die auf den Hebelarm 26 wirkende Federkraft bewirkt dabei, dass die Rastzunge 50 an der unteren Kantenfläche 68 entlang gleitet und die Rastzunge 50 auf die Rückseite 66 der Arretiereinrichtung 42 führt. Da hier keine Rastnasen vorgesehen sind, kann der Hebelarm 26 ungehindert in die erste Endposition zurückschwenken.

In Fig. 3 ist der Hebelarm 26 in schematischer vergrößerter Darstellung gezeigt. Hierbei ist zu erkennen, dass der Hebelarm 26 ein Langloch 70 aufweist, durch das das Seitenrohr 14 hindurchläuft. Darüber hinaus weist der Hebelarm 26 eine Griffmulde 72 auf, die an der dargestellten Kante 74 endet.

Fig. 3 lässt noch erkennen, dass das Seitenrohr 14 zwei seitlich angebrachte Stege 76 besitzt, die in einer (bezogen auf das Seitenrohr 14) radialen Ebene liegen. Die Abmessung in radialer Richtung (bezüglich des Seitenrohrs 14) ist so gewählt, dass die Stege 76 kurz vor dem jeweiligen Rand des Langlochs 70 enden. Mit Hilfe dieser Stege 76 soll verhindert werden, dass sich der Hebelarm 26 senkrecht zur Längsachse bewegen kann. Eine solche senkrechte (parallel zur Drehachse 28) Bewegung hätte nämlich unter Umständen zur Folge, dass sich die Rastzunge 50 aus einer Rastnase 48 löst, da sich Rastnase 48 und Rastzunge 50 in diese Richtung (senkrecht zur Längsachse des Ventilkörpers) erstrecken. Die Stege 76 dienen folglich der Führung des Hebelarms 26 in einer Drehebene. Wie sich aus Fig. 1 ergibt, verlaufen die Stege 76 längs zum Seitenrohr 14 in Richtung des Ventilkörpers 12.

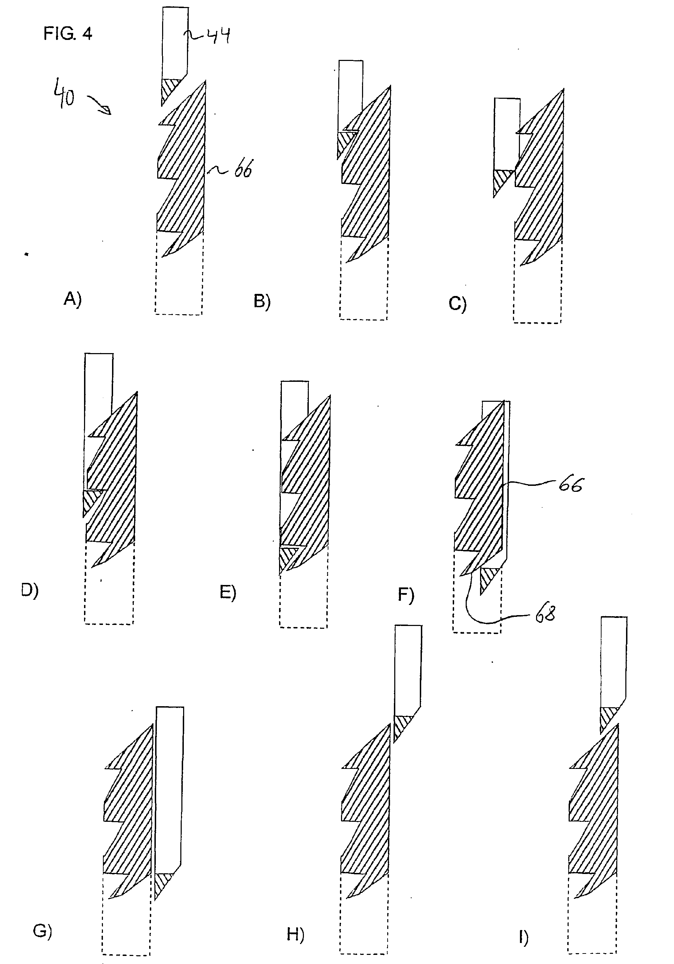

In den Fig. 4A bis 4I ist die Funktionsweise des Rastmechanismus 40 nochmals näher erläutert. Zur Vereinfachung wurden jedoch die in Fig. 2 verwendeten Bezugszeichen nicht nochmals angegeben. Die Reihenfolge der dargestellten Stellungen in den Fig. 4A bis 4I entspricht dabei einer Bewegung des Hebelarms 26 aus der ersten oberen Endposition in die zweite untere Endposition 34 und wieder zurück in die erste Endposition. Fig. 4B zeigt die Rastzungeneinrichtung 44 in einer ersten Raststellung, in Fig. 4D in einer zweiten Raststellung und in Fig. 4E in einer dritten Raststellung. Wird der Hebelarm 26 weiter gegen die Federkraft 30 in Richtung des Ventilkörpers 12 gedrückt, gelangt die Rastzunge 50 auf die Kantenfläche 68, wie in Fig. 4F dargestellt, und gleitet beim Loslassen des Hebelarms 26 an dieser entlang auf die Rückseite 66 der Arretiereinrichtung 42. An dieser Rückseite 66 gleitet dann die Rastzunge 50 nach oben zurück in die erste Endposition, wie in den Fig. 4G bis 4I dargestellt.

Da der Hebelarm 26 und damit die Rastzunge 50 nicht in Richtung der Drehachse 28 bewegt werden kann, was durch die Stege 76 verhindert wird, kann der Hebelarm 26 aus jeder der Raststellungen nur durch eine Bewegung in die zweite Endposition 34 zurück in die erste Endposition gebracht werden. Ein direktes Zurückbewegen aus einer Raststellung in die erste Endposition ist folglich nicht möglich.

Da der Benutzer der Vorrichtung 10 zum Rasten und Lösen des Hebelarms demnach nur eine Bewegung des Hebelarms 26 durchführen muss, ist die Bedienung besonders einfach.

## Patentansprüche

1. Vorrichtung zur Handhabung eines Katheters mit
- einem länglichen Ventilkörper (12),
- einem Hebelarm (26), der über ein Federelement (30) vorgespannt ist und an dem Ventilkörper (12) schwenkbar angebracht ist, um aus einer ersten Endposition gegen die Vorspannungskraft des Federelements in eine zweite Endposition (34) schwenkbar zu sein,
- einem im Ventilkörper (12) aufgenommenen Druckkolben (24), der über den Hebelarm (26) in Längsrichtung bewegbar ist, um ein Dichtungselement im Ventilkörper (12) bei einer Bewegung des Hebelarms (26) in Richtung der zweiten Endposition (34) zu öffnen, und
- einem Rastmechanismus (40), der eine Arretiereinrichtung (42) und eine damit zusammenwirkende Rastzunge (50) aufweist, um den Hebelarm (26) in zumindest einer Raststellung zu halten,
**dadurch gekennzeichnet, dass** der Rastmechanismus (40) so ausgebildet ist, dass die Arretiereinrichtung (42) und die Rastzunge (50) bei Erreichen der zweiten Endposition (34) des Hebelarms (26) außer Eingriff gelangen, so dass das Federelement (30) den Hebelarm (26) zurück in die erste Endposition führt.

2. Vorrichtung nach Anspruch 1, **dadurch gekennzeichnet, dass** die Arretiereinrichtung (42) mehrere Rastnasen (48) aufweist, die entlang einer konzentrischen Linie um den Drehpunkt des Hebelarms (26) angeordnet sind, um mehrere Raststellungen des Hebelarms (26) zwischen den beiden Endpositionen zu definieren.

3. Vorrichtung nach Anspruch 1 oder 2, **dadurch gekennzeichnet, dass** der dem Ventilkörper (12) zugewandten Rastnase (48) eine schräg verlaufende Führungsfläche folgt, um bei Erreichen der zweiten Endposition (34) des Hebelarms (26) die Rastzunge (50) auf die der Rastnase (48) abgewandte Seite zu führen, so dass die Rastzunge (50) bei der Rückführung des Hebelarms (26) in die erste Endposition nicht mit den Rastnasen (48) in Eingriff gelangen kann.

4. Vorrichtung nach Anspruch 2 oder 3, **dadurch gekennzeichnet, dass** sich die Rastzunge (50) und die Rastnasen (48) quer zur Längsrichtung des Ventilkörpers (12) erstrecken.

5. Vorrichtung nach einem der vorhergehenden Ansprüche, **dadurch gekennzeichnet, dass** der Hebelarm (26) ein Langloch (70) aufweist, durch das sich ein vom Ventilkörper (12) abgehendes Seitenrohr (14) hindurch erstreckt.

6. Vorrichtung nach einem der vorhergehenden Ansprüche, **dadurch gekennzeichnet, dass** der Ventilkörper (12) und/oder der Hebelarm (26) als Spritzgießteil ausgebildet sind.

7. Vorrichtung nach Anspruch 6, **dadurch gekennzeichnet, dass** der Ventilkörper (12) und die Arretiereinrichtung (42) a**ls** einstückiges Bauteil ausgebildet sind.

## Claims

1. Device for handling a catheter with
- an elongate valve body (12),
- a lever arm (26) which is prestressed by means of a spring element (30) and mounted pivotably on the valve body (12) so as to be pivotable from a first end position into a second end position (34) counter to the prestressing force of the spring element,
- a pressure piston (24) which is received in the valve body (12) and can be moved in the longitudinal direction by means of the lever arm (26) in order to open a sealing element in the valve body (12) when the lever arm (26) is moved in the direction of the second end position (34), and
- a catch mechanism (40) which has an arresting arrangement (42) and a catch tongue (50) interacting therewith in order to hold the lever arm (26) in at least one catching position,
**characterized in that** the catch mechanism (40) is designed in such a way that the arresting arrangement (42) and the catch tongue (50) come out of engagement when the second end position (34) of the lever arm (26) is reached, so that the spring element (30) guides the lever arm (26) back into the first end position.

2. Device according to Claim 1, **characterized in that** the arresting arrangement (42) has a number of catch noses (48) which are arranged along a concentric line around the fulcrum of the lever arm (26) in order to define a number of catching positions of the lever arm (26) between the two end positions.

3. Device according to Claim 1 or 2, **characterized in that** the catch nose (48) facing the valve body (12) follows a guide surface extending at an angle in order, when the second end position (34) of the lever arm (26) is reached, to guide the catch tongue (50) onto the side facing away from the catch nose (48), so that the catch tongue (50) cannot enter into engagement with the catch noses (48) when the lever arm (26) is returned into the first end position.

4. Device according to Claim 2 or 3, **characterized in that** the catch tongue (50) and the catch noses (48) extend transversely to the longitudinal direction of the valve body (12).

5. Device according to one of the preceding claims, **characterized in that** the lever arm (26) has an elongated hole (70), through which a lateral tube (14) starting from the valve body (12) extends.

6. Device according to one of the preceding claims, **characterized in that** the valve body (12) and/or the lever arm (26) are designed as an injection-molded part.

7. Device according to Claim 6, **characterized in that** the valve body (12) and the arresting arrangement (42) are designed as a one-piece component.

## Revendications

1. Dispositif de manipulation d'un cathéter comportant
- un corps de valve (12) allongé,
- un bras de levier (26), qui est précontraint par le biais d'un élément formant ressort (30) et est monté de manière pivotante sur le corps de valve (12) pour pouvoir être pivoté d'une première position d'extrémité contre la force de précontrainte de l'élément formant ressort à une deuxième position d'extrémité (34),
- un piston de compression (24) logé dans le corps de valve (12), lequel piston de compression est mobile dans le sens longitudinal par le biais du bras de levier (26) pour ouvrir un élément d'étanchéité dans le corps de valve (12) lors d'un mouvement du bras de levier (26) dans le sens de la deuxième position d'extrémité (34), et
- un mécanisme d'encliquetage (40), qui présente un équipement d'arrêt (42) et une languette d'encliquetage (50) coopérant avec celui-ci, pour maintenir le bras de levier (26) dans au moins une position d'encliquetage,
**caractérisé en ce que** le mécanisme d'encliquetage (40) est réalisé de telle sorte que l'équipement d'arrêt (42) et la languette d'encliquetage (50) se mettent hors prise lorsque le bras de levier (26) atteint la deuxième position d'extrémité (34) de sorte que l'élément formant ressort (30) ramène le bras de levier (26) dans la première position d'extrémité.

2. Dispositif selon la revendication 1, **caractérisé en ce que** l'équipement d'arrêt (42) présente plusieurs nez d'encliquetage (48), qui sont agencés le long d'une ligne concentrique autour du point de rotation du bras de levier (26) pour définir plusieurs positions d'encliquetage du bras de levier (26) entre les deux positions d'extrémité.

3. Dispositif selon la revendication 1 ou 2, **caractérisé en ce qu'**une surface de guidage s'étendant en biais suit le nez d'encliquetage (48) tourné vers le corps de valve (12) pour guider la languette d'encliquetage (50) sur le côté opposé au nez d'encliquetage (48) lorsque le bras de levier (26) atteint la deuxième position d'extrémité (34) de sorte que la languette d'encliquetage (50) ne peut pas entrer en prise avec les nez d'encliquetage (48) lorsque le bras de levage (26) est ramené dans la première position d'extrémité.

4. Dispositif selon la revendication 2 ou 3, **caractérisé en ce que** la languette d'encliquetage (50) et les nez d'encliquetage (48) s'étendent transversalement au sens longitudinal du corps de valve (12).

5. Dispositif selon l'une quelconque des revendications précédentes, **caractérisé en ce que** le bras de levier (26) présente un trou oblong (70) à travers lequel s'étend un tube latéral (14) partant du corps de valve (12).

6. Dispositif selon l'une quelconque des revendications précédentes, **caractérisé en ce que** le corps de valve (12) et/ou le bras de levier (26) sont réalisés sous forme de pièce moulée par injection.

7. Dispositif selon la revendication 6, **caractérisé en ce que** le corps de valve (12) et l'équipement d'arrêt (42) sont réalisés sous la forme d'un élément d'un seul tenant.
